# EUROPEAN PATENT APPLICATION

(11) **EP 4 692 778 A1**
(43) Date of publication of application: **11.02.2026**
(21) Application number: 24780407.3
(22) Date of filing: 27.03.2024
(51) Int. Cl.: G01N 27/62, C12Q 1/68, G01N 33/50

(54) **METHOD FOR DETECTING PYROPHOSPHORIC ACID**

(30) Priority: 31.03.2023 JP 2023058095
(71) Applicant: Denka Company Limited, Tokyo 103-8338 (JP)
(72) Inventor: KODAMA, Keisuke, Tokyo 103-8338 (JP); KUBO, Hiroshi, Tokyo 103-8338 (JP)
(74) Representative: Müller-Boré & Partner Patentanwälte PartG mbB
(86) International application number: PCT/JP2024/012156
(87) International publication number: WO 2024/204301

(57) **Abstract**

The present invention provides a method for detecting pyrophosphoric acid in a sample, wherein the method comprises a step of ionizing pyrophosphoric acid derived from a phosphoric acid compound in a sample and determining mass of this pyrophosphoric acid using a prescribed mass spectrometer.

## Description

### Technical Field

The present invention, considered broadly, relates to, inter alia, a method for detecting pyrophosphoric acid.

### Background Art

DNA polymerase, which is an enzyme that synthesizes DNA by catalyzing nucleotide transfer reactions, incorporates a dNTP that is complementary to the base in a template strand, and forms a phosphodiester bond between the hydroxyl group at the 3'-terminal of the primer strand and the α-phosphorus atom of the dNTP, and moreover cleaves the bond between the α-phosphorus atom and the β-phosphorus atom of the dNTP. As a result, the DNA strand is extended by one base and pyrophosphoric acid is released. Attempts have been made to analyze nucleic acids, e.g., DNA and RNA, by utilizing the pyrophosphoric acid generated during the course of such base extension reactions.

Patent Literature 1 discloses a method for quantifying nucleic acids by using a technique (a colorimetric method) for indirectly detecting pyrophosphoric acid, in which the pyrophosphoric acid is converted into ATP by sulfurylase and the luminescence generated by the luciferase-mediated action of the ATP on luciferin is detected.

### Citation List

### Patent Literature

Patent Literature 1: Patent Publication JP-A-2004-187603

### Summary of Invention

### Technical Problem

Noise is a problem in systems that detect pyrophosphoric acid via luminescence signals, such as colorimetric methods. In nucleic acid extension reactions, if paired bases (A and T or G and C) do not react smoothly with the DNA strand to be read, a delay in DNA synthesis may be produced, resulting in a lag in the reaction. In this case, light is emitted with a timing different from the timing that should occur for the original reaction, and as a result light emission generated by the inability to bring about the appropriate synthesis reaction is detected as noise in colorimetric methods.

In addition, the remaining dNTP that is unable to react is usually degraded by apyrase; however, when such a base decomposition is unsuccessful and base remains in the reaction system, the residual base may be admixed in the synthesis step for the next base read and the luminescence emitted from the incorrect base may end up as noise in a colorimetric method.

### Solution to Problem

The present inventors discovered that pyrophosphoric acid can be directly detected using matrix-assisted laser desorption/ionization mass spectrometry (MALDI/MS) and thereby achieved the present invention.

That is, the present application encompasses the following invention.
[1] A method for detecting pyrophosphoric acid in a sample, the method comprising:
   a step of ionizing pyrophosphoric acid derived from a phosphoric acid compound in a sample and determining mass of this pyrophosphoric acid by matrix-assisted laser desorption/ionization mass spectrometry (MALDI/MS).
[2] The method according to [1], wherein the ionization is carried out in the presence of a matrix.
[3] The method according to [2], wherein the matrix comprises one or a plurality of cyclic compounds.
[4] The method according to [3], wherein the cyclic compound has a heterocycle.
[5] The method according to [4], wherein a heteroatom constituting the heterocycle is nitrogen.
[6] The compound according to any of [3] to [5], wherein the cyclic compound is a compound selected from the group consisting of trans-3-indoleacrylic acid (IAA), 9-aminoacridine (9-AA), 9H-pyrido[3,4-b]indole, and 1-methyl-7-methoxy-3,4-dihydro-β-carboline.
[7] The method according to [2], wherein the matrix further comprises an ionization aid.
[8] The method according to any of [1] to [7], wherein the sample comprises a nucleic acid as the phosphoric acid compound and a polymerase, and wherein the pyrophosphoric acid released from the nucleic acid by the polymerase is detected.
[9] The method according to [8], wherein ionization is carried out in the presence of manganese chloride and/or cobalt chloride.

### Advantageous Effect of Invention

The present invention avoids the noise problem caused by colorimetric methods and thereby enables a more accurate analysis of nucleic acids.

A decline in peak intensity may be observed when the pyrophosphoric acid liberated by an extension reaction mediated by, e.g., DNA polymerase, is measured using a mass spectrometer. The present inventors hypothesized that chloride ions present in the reaction solution exert an influence in the form of an ion suppression effect when pyrophosphoric acid is measured by MALDI-TOF/MS. Such an ion suppression can be avoided by carrying out the detection of pyrophosphoric acid in the presence of manganese chloride and/or cobalt chloride.

The present invention also makes possible the analysis of nucleic acids by measuring the mass of the released pyrophosphoric acid that is produced in a base extension reaction. Since mass spectrometry exhibits a higher sensitivity than conventional methods of analysis that use a luminescence signal as an indicator, highly accurate results can then be provided when nucleic acid sequence determination is carried out based on the mass of the released pyrophosphoric acid. The present invention is also useful for the detection of a target nucleic acid contained in very small specimens.

### Brief Description of Drawings

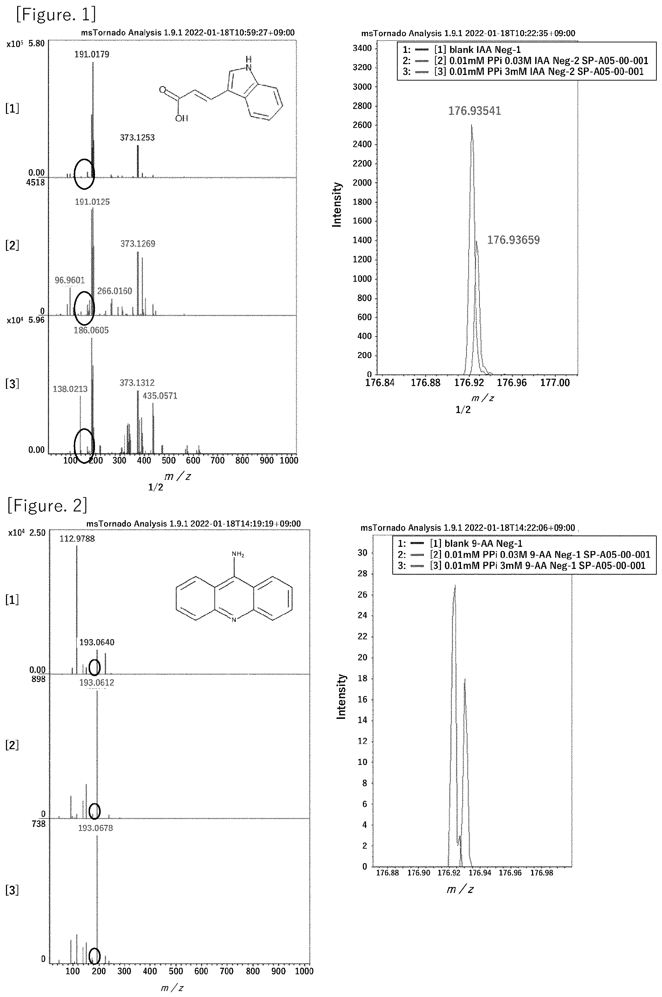

[Figure 1] Figure 1 gives mass spectra (left side) obtained using trans-3-indoleacrylic acid (IAA) for the matrix and also gives an enlargement (right side) in the vicinity of a mass/charge ratio (m/z) = 177. The mass spectra on the left are for IAA + DAC (upper), 0.01 mM sodium pyrophosphate + 30 mM IAA + DAC (middle), and 0.01 mM sodium pyrophosphate + 3 mM IAA + DAC (bottom).
[Figure 2] Figure 2 gives mass spectra (left side) obtained using 9-aminoacridine (9-AA) for the matrix and also gives an enlargement (right side) in the vicinity of a mass/charge ratio (m/z) = 177. The mass spectra on the left are for 9-AA (upper), 0.01 mM sodium pyrophosphate + 30 mM 9-AA (middle), and 0.01 mM sodium pyrophosphate + 3 mM 9-AA (bottom).

### Description of Embodiments

Embodiments of the present invention ("the present embodiments" hereafter) are described in the following, but the scope of the invention should not be construed as being limited to the following embodiments.

### (Analytic method)

In a first aspect there is provided a method for detecting the pyrophosphoric acid in a sample, wherein the method comprises a step of ionizing the pyrophosphoric acid derived from a phosphoric acid compound in the sample and determining the mass of this pyrophosphoric acid using a prescribed mass spectrometric method.

As used in the present Disclosure, "phosphoric acid compound" is a compound that has one or a plurality of phosphoric acid groups in its structure and is not particularly limited as long as it comprises a compound that can release pyrophosphoric acid upon decomposition, such as the pyrophosphoric acid produced as a result of the cleavage of the bond between the α-phosphorus atom and the β-phosphorus atom of a deoxynucleoside triphosphate (dNTP). The phosphoric acid compound may be an organic or inorganic species. Nucleotides are examples of organophosphoric acid compounds.

The nucleotide may be a mononucleotide or an oligonucleotide. The mononucleotides can be exemplified by nucleotides (including, e.g., nucleotide monophosphates, nucleotide diphosphates, and nucleotide triphosphates) and deoxynucleotides (including, e.g., deoxynucleotide monophosphates, deoxynucleotide diphosphates, and deoxynucleotide triphosphates) and mononucleotides of nucleosides, deoxynucleosides, and so forth. The length of the oligonucleotide is not limited.

Nucleotides generally comprise a sugar (e.g., ribose or deoxyribose), a base, and at least one phosphoric acid group. The "base" denotes a compound, referred to as a nucleobase, that comprises a nitrogenous base, a pentose sugar (ribose or deoxyribose), and at least one phosphoric acid group and that constitutes nucleic acids such as ribonucleotides, deoxyribonucleotides, nucleosides, and modified nucleotides.

The base may be adenine (A), guanine (G), thymine (T), cytosine (C), or uracil (U). The base is not limited to these and encompasses any natural or artificial nucleobase. Specific examples of bases other than the above bases include modified purine bases, for example, hypoxanthine, xanthine, uric acid, 7-methylguanine, 2,6-diaminopurine, 6,8-diaminopurine, N6-methyladenine, 7-deazaxanthine, and 7-deazaguanine; and modified pyrimidine bases, for example, 5,6-dihydrouracil, 5-methylcytosine, 5-hydroxymethylcytosine, N4,N4-ethanocytosine, 5-fluorouracil, and 5-bromouracil.

The nucleotides can be exemplified by the following, although there is no limitation thereto: adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine monophosphate (dCMP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), and deoxyuridine triphosphate (dUTP).

Ribonucleotides can be exemplified by ATP, UTP, CTP, GTP, ADP, UDP, CDP, GDP, AMP, UMP, CMP, and GMP, while deoxyribonucleotides can be exemplified by dATP, dTTP, dCTP, dGTP, dADP, dTDP, dCDP, dGDP, dAMP, dTMP, dCMP, and dGMP, although there is no limitation to the preceding.

The nucleotides may include deoxyribonucleotides, modified deoxyribonucleotides, ribonucleotides, modified ribonucleotides, peptide nucleotides, modified peptide nucleotides, modified phosphate-sugar backbone nucleotides, and mixtures of the preceding.

In an embodiment, the nucleotides are deoxynucleotide triphosphates (dNTPs).

The dNTPs are one or a plurality of selections from the group consisting of dATP, dGTP, dCTP, and dTTP, and may be, for example, a mixture of dATP, dGTP, dCTP, and dTTP. The mixing ratio for the dATP, dGTP, dCTP, and dTTP may be freely selected, and the mixture may be an equimolar mixture.

In an embodiment, the nucleotides are polynucleotides or analogs thereof.

The phosphoric acid compound may be in the form of a polynucleotide, for example, a nucleic acid such as a deoxyribonucleic acid (DNA), a ribonucleic acid (RNA), or their analogs. The nucleic acid can include single-stranded, double-stranded, or both single-stranded and double-stranded sequences. The nucleic acid molecule may be derived from double-stranded DNA (dsDNA) (e.g., genomic DNA, amplification products provided by, e.g., PCR, and so forth) or single-stranded DNA (ssDNA) or RNA. Nucleic acids that function as templates in base extension reactions are preferred.

The nucleic acid molecules include naturally occurring nucleic acid molecules, for example, genomic DNA, genomic DNA fragments, exons, introns, RNA, ribozymes, and so forth, and synthetic nucleic acid molecules, for example, complementary DNA (cDNA), recombinant polynucleotides, vectors, probes, primers, and so forth.

The RNA may be a coding RNA, e.g., messenger RNA (mRNA), or the RNA may be a non-coding RNA. Non-coding RNA includes transfer RNA (tRNA), ribosomal RNA (rRNA), microRNA (miRNA), siRNA (small interfering RNA), piRNA (PIWI-interacting RNA), small nucleolar ribonucleoprotein (snoRNP), small nuclear RNA (snRNA), extracellular RNA (ExRNA), and so forth.

The synthetic DNAs and RNAs can be artificially produced, based on a prescribed base sequence (natural sequence or nonnatural sequence), using, for example, an automatic nucleic acid synthesizer.

Pyrophosphoric acid is a compound represented by the chemical formula (HO)₂P(O)OP(O)(OH)₂ and is also called diphosphoric acid. Pyrophosphoric acid derived from a phosphoric acid compound may be, for example, the pyrophosphoric acid generated during amplification of a nucleic acid molecule, e.g., DNA or RNA, using any nucleic acid amplification method, or the pyrophosphoric acid synthesized in a biological sample as a product of a nucleic acid synthesis reaction mediated by DNA polymerase or RNA polymerase.

The phosphoric acid compound-containing sample should comprise a phosphoric acid compound or should be suspected of comprising a phosphoric acid compound, but is not otherwise particularly limited, and can be exemplified by bodily fluids such as blood, serum, plasma, urine, stool, saliva, sputum, tissue fluid, cerebrospinal fluid, swab fluids, and so forth, originating from humans or non-humans, and their dilutions, wherein blood, serum, plasma, urine, stool, cerebrospinal fluid, and dilutions thereof are preferred. The non-humans include non-human animals as well as plants, insects, microorganisms, viruses, and so forth. The non-human animals include mammals such as the mouse, rat, hamster, guinea pig, rabbit, cat, dog, pig, and monkey. Samples may be collected from various environments such as food, beverages, soil, and so forth.

The sample may comprise a chemically synthesized phosphoric acid compound.

As used in the present Description, "mass spectrometry" refers to any method in which a substance is converted using various ionization methods into an atomic or molecular ion and the mass and number thereof are measured. Mass spectrometers are classified in accordance with the mass spectrometric section into, for example, quadrupole types, ion trap types, magnetic field types, and time-of-flight types. Mass spectrometry is classified, for example, into the following in accordance with the ion source (ionization section): electron ionization (EI) methods, chemical ionization (CI) methods, field desorption (FD) methods, fast atom bombardment (FAB) methods, matrix-assisted laser desorption/ionization (MALDI) methods, electrospray ionization (ESI) methods, atmospheric pressure chemical ionization (APCI) methods, and inductively coupled plasma (ICP) methods; however, any method that can measure pyrophosphoric acid may be used.

Mass spectrometers that employ columns, such as liquid chromatography-mass spectrometry (LC-MS), require lengthy times for measurement and thus have reduced throughputs, and, as a consequence, mass spectrometers that do not use columns are preferred. Also, mild ionization instrumentation is preferred in order to avoid fragmentation of the pyrophosphoric acid.

In an embodiment, ionization is performed in the presence of a matrix using matrix-assisted laser desorption/ionization mass spectrometry (MALDI/MS).

A sample introduced into a mass spectrometer is ionized by the ion source, yielding ions present in the gas phase. These ions exhibit different mobilities depending on their mass/charge ratio (m/z), and as a result they are separated in the analyzer based on the various principles and are detected using a detector. The matrix-assisted laser desorption/ionization (MALDI) technique is known as one of the ionization techniques used in mass spectrometry. In the MALDI technique, a laser beam is briefly irradiated onto the sample, causing an instantaneous vaporization of the sample and thereby ionizing the molecules of the substance to be measured in the sample.

The sample impinged by the laser via the matrix is ionized in the mass spectrometer. A sample comprising pyrophosphoric acid or a salt thereof to be ionized, or comprising a phosphoric acid compound that is the precursor therefor, is prepared as appropriate in advance in accordance with the mass spectrometer to be used.

The solvent constituting the sample is not particularly limited, and water, an organic solvent, or a mixture thereof is used. From the standpoint of further promoting ionization, an ionization aid may be added to the sample as appropriate. Such an ionization aid is not particularly limited, and, for example, a known ionization aid such as trifluoroacetic acid or citric acid or a salt of the preceding, for example, sodium trifluoroacetate, diammonium hydrogen citrate, and so forth, can be used as appropriate.

In the MALDI technique, a solution comprising the phosphoric acid compound is mixed with the matrix, and, after optionally mixing with an ionization aid, this is coated on a sample plate and the sample is prepared by removing the solvent by, for example, drying.

The detected fragments and peak intensities will change depending on the matrix, and the spectrum will also change depending on the presence/absence of an ionization aid, but the person skilled in the art can adjust the ion intensity, peak intensity, and spectrum as appropriate. In the case of MALDI, the peak intensity can be increased by changing the settings at the instrument, for example, by increasing the laser intensity. The spectrum can otherwise also be adjusted by adjusting the delay time (the time interval for counting ions that have reached the detector).

As used in this Description, "ion intensity" refers to the amount of ions detected by a mass spectrometer. Ion intensity corresponds to the vertical axis of a mass spectrum. As used in this Description, "peak intensity" refers to the amount of ions having each m/z (a dimensionless quantity obtained by splitting up the ion mass by unified atomic mass units and dividing by the amount of charge on the ion) as generated by ionization of an analyte. The ion intensity corresponds to the peak height (peak area) in a mass spectrum.

Any matrix can be used that itself undergoes ionization upon absorbing the laser and supplies a proton or anion to the sample; however, the matrix is preferably a cyclic compound composed of one or a plurality of rings. The cyclic compound is preferably a compound composed of a plurality of rings, in which the hydrogen atoms on the individual rings are not substituted by the carboxyl group or hydroxyl group. The cyclic compound preferably comprises a heterocyclic ring, and the heteroatom is particularly preferably nitrogen.

In an embodiment, the cyclic compound is selected from the group consisting of trans-3-indoleacrylic acid (IAA), 9-aminoacridine (9-AA), 9H-pyrido[3,4-b]indole (norharmane), and 1-methyl-7-methoxy-3,4-dihydro-β-carboline (harmaline).

The pyrophosphoric acid is preferably detected in the form of unfragmented P₂O₇, but fragmentation is facilitated when a matrix is used that comprises many hydroxyl groups or carboxylic acid groups. It is therefore preferable that the matrix not comprise 2,5-dihydroxybenzoic acid (DHB), 4-hydroxycinnamic acid (CHCA), 3-hydroxypicolinic acid (HPA), or 2,4,6-trihydroxyacetophenone (THAP).

The matrix is prepared such that the matrix is present in an amount that is approximately 100X to 10,000X, as the molar ratio, with respect to the phosphoric acid compound. The molar ratio of the matrix to the phosphoric acid compound is preferably 1,000X to 5,000X and is particularly preferably 2,000X to 4,000X.

When the phosphoric acid compound is a nucleic acid, the pyrophosphoric acid released from the nucleic acid by a polymerase may be detected. The polymerase is a nucleic acid synthase that performs complementary strand synthesis (base extension reaction) using nucleic acid as a template, and pyrophosphoric acid is generated in the process of adding a nucleotide to the end of the nucleic acid. Any enzyme can be used - even if it is not referred to as a polymerase such as a DNA polymerase or an RNA polymerase - that can generate pyrophosphoric acid from a nucleic acid in the process of performing complementary strand synthesis (base extension reaction) using the nucleic acid as a template. Such enzymes can be exemplified by reverse transcriptase and terminal deoxynucleotidyl transferase. The reaction with the particular enzyme can be carried under any known conditions.

The chloride ion, which is essential for base extension reactions, may affect the detection of pyrophosphoric acid via ion suppression. In order to avoid this ion suppression, pyrophosphoric acid detection is preferably carried out in the presence of manganese chloride or cobalt chloride. The concentrations of manganese chloride and cobalt chloride are adjusted as appropriate depending on the concentration of the nucleic acid. For example, when the dNTP concentration is 1 to 3 µM, the manganese chloride and cobalt chloride may have any concentration, but the ddNTP concentration is preferably more than 20 µM and is more preferably at least 100 µM, for example, at least 110 µM, at least 120 µM, at least 130 µM, at least 140 µM, at least 150 µM, at least 160 µM, at least 170 µM, at least 180 µM, at least 190 µM, or at least 200 µM. When the dNTP concentration is 10 to 30 µM, the concentrations of manganese chloride and cobalt chloride are adjusted as appropriate within a concentration range of 0.1 µM to 300 µM and preferably 1 to 200 µM. In this case, the ddNTP concentration is preferably more than 20 µM.

The mass spectrometric procedure can include required steps other than the pyrophosphoric acid ionization step and the step of measuring the mass of the ionized molecules.

The detection of pyrophosphoric acid using a mass spectrometer can be applied to various objectives. For example, the analysis of nucleic acids, such as sequence determination, can be carried out by measuring the masses of pyrophosphoric acids having different masses that are released from phosphoric acid compounds having different molecular weights due to isotopes with different masses.

### (Sequence determination method)

A second aspect provides a method for analyzing nucleic acids, wherein the method comprises:
1) a step of bringing a target nucleic acid into contact, under conditions that support a base extension reaction, with a polymerase and a plurality of substrates in which the phosphoric acid groups have been labeled with isotopes having different masses from each other; and
2) a step of measuring the mass of the liberated pyrophosphoric acid that is produced by base incorporation.

The "nucleic acid" is as has already been described above. The nucleic acid to be analyzed may be single-stranded or double-stranded, but a double-stranded target nucleic acid is adjusted to a single strand by denaturation prior to contact with the polymerase. The target nucleic acid may be amplified prior to the reaction and may be immobilized on, for example, a support.

The nucleic acid that is the target for sequence determination (target nucleic acid) may be pretreated prior to the base extension reaction. For example, in order to detect the presence/absence of DNA methylation, the target nucleic acid may be subjected to a bisulfite treatment to convert cytosine that is not methylated (unmethylated cytosine) to uracil.

The target nucleic acid may be derived from a human or non-human animal, a plant, an insect, a microorganism, or a virus, or from a biological sample derived from the preceding, for example, blood, serum, plasma, saliva, tissue, cells, and so forth, or may be chemically synthesized. The non-human animals include, for example, mammals such as the mouse, rat, hamster, guinea pig, rabbit, cat, dog, pig, and monkey.

As used in this Description, "target nucleic acid" refers to a nucleic acid that is the target of the analysis and is one or more species of nucleic acid that may function as a template for a base extension reaction by a polymerase. The present invention can also be applied to target nucleic acids that have regions of consecutive identical bases or regions with complex mutations. Such regions include homopolymer sequences such as AAAA, which occur frequently in the human genome, and sequences that have received epigenetic modification, such as methylated cytosine in CpG islands.

The target nucleic acid can be primed with a primer. This primer is an oligonucleotide provided with a 5'-terminal and a 3'-terminal having a sequence complementary to at least a portion of the target nucleic acid.

As used in this Description, "polymerase" denotes a nucleic acid synthase that performs complementary strand synthesis (base extension reaction) using a nucleic acid as a template, for example, a DNA polymerase, RNA polymerase, reverse transcriptase, and so forth. The polymerase has one or more active sites where nucleotide binding and/or a catalytic action in nucleotide polymerization can occur. The polymerase extends bases from the 3'-terminal of the primer bound to the target nucleic acid. A known polymerase, e.g., a commercially available product, can be used as the polymerase.

The DNA polymerase can be exemplified by, but is not limited to, bacterial DNA polymerases, eukaryotic DNA polymerases, archaebacterial DNA polymerases, viral DNA polymerases, and phage DNA polymerases.

The RNA polymerase can be exemplified by, but is not limited to, viral RNA polymerases, eukaryotic RNA polymerases, and archaebacterial RNA polymerases.

The target nucleic acid may be isolated prior to contact with, e.g., the polymerase, or may be contained in a specimen. This specimen may comprise molecules other than the target nucleic acid. The specimen is not particularly limited as long as it is known to comprise the target nucleic acid or is suspected of comprising the target nucleic acid, and it may be a biological sample from which nucleic acid as described above is derived, or may be a dilution, concentrate, or culture of such a biological sample. The specimen may be treated by, for example, formalin fixation, FFPE embedding, and so forth. The specimen may be cancer tissue collected by, e.g., biopsy. In this case, the specimen will typically comprise cancer cells and normal cells, and the cancer cell abundance ratio can be evaluated by making the target nucleic acid a nucleic acid present in only the cancer cells or normal cells.

The base incorporated in the base extension reaction may also be referred to as a "substrate" in this Description. The phosphoric acid group constituting the substrate is labeled with an isotope. As used in this Description, "isotope" refers to an isotope that has the same atomic number while having a different number of neutrons, and such isotopes include stable isotopes and radioisotopes. There are various examples of stable isotopes, and, among them, stable oxygen isotopes (¹⁶O, ¹⁷O, ¹⁸O) and stable sulfur isotopes (³²S, ³³S, ³⁴S, ³⁶S) are preferred. Other stable isotopes are, for example, ²H, ¹³C, and ¹⁵N.

To explain isotope labeling using the example of the use of DNA as the target nucleic acid, the four types of bases to be incorporated into the target DNA are prepared prior to the base extension reaction, and these are labeled with isotopes so that the masses of the pyrophosphoric acid released from these bases are different from each other. The target nucleic acid can be analyzed by measuring the mass of the pyrophosphoric acid each time it is released in the base extension reaction. Since pyrophosphoric acid is released in an amount equimolar with the extended base, the mass difference for the individual bases is identified at each single base extension and the proportion of gene mutations can be analyzed from the quantity ratio thereof.

Since the detection of the incorporated substrate or the released pyrophosphoric acid produced therefrom is carried out using mass spectrometry rather than luminescence or fluorescence, the substrate and pyrophosphoric acid constituting it are preferably not modified other than by isotope labeling. The present invention differs in this respect from existing sequence determination methods, e.g., Sanger sequencing, next-generation sequencing (NGS), and pyrosequencing. However, sites other than the phosphoric acid group may be further labeled for purposes other than the detection of pyrophosphoric acid. For example, the 3'-OH group of the base may be modified.

As an auxiliary, the substrate incorporated during an amplification reaction process can also be labeled with, e.g., a fluorescent substance or luminescent substance other than an isotope. Separately from the mass spectrometry, the amplification product incorporating the labeled substrate can also be monitored as appropriate in accordance with the label.

As used in this Description, a "base extension reaction" denotes a reaction in which a complementary strand for a target nucleic acid is synthesized by a polymerase. Base extension reactions typically proceed by repeating the following individual reactions: thermal denaturation of the base sequence, annealing of the primer, and synthesis of the complementary strand by the polymerase. Conditions such as temperature control in the individual reactions can be set as appropriate by the person skilled in the art who understands nucleic acid amplification reactions such as PCR. In addition to the PCR method, such nucleic acid amplification reactions can be exemplified by the LAMP method, the LCR method, the TMA method, the SDA method, the RT-PCR method, the RT-LAMP method, the NASBA method, the TRC method, and the TMA method. The base extension reaction is carried out using a nucleic acid amplification device such as a thermal cycler.

In addition to the target nucleic acid, polymerase, and labeled substrate, the following are added as appropriate to the reaction solution for the base extension reaction: primer; divalent metal ion selected from the group consisting of rhodium, calcium, magnesium, cobalt, nickel, manganese, zinc, cadmium, and chromium; buffer; and so forth. The target nucleic acid and the polymerase may be immobilized on a solid surface such as a support.

The support is not particularly limited as long as it is a solid support as generally used for immobilizing nucleic acids and polymerases, but the support is preferably a solid support that is insoluble in water and does not melt during thermal denaturation. Such materials can be exemplified by metals such as gold, silver, copper, aluminum, tungsten, molybdenum, chromium, platinum, titanium, and nickel; alloys such as stainless steel, Hastelloy, Inconel, Monel, and duralumin; silicon; glass materials such as glass, quartz glass, fused quartz, synthetic quartz, alumina, sapphire, ceramics, forsterite, and photosensitive glass; plastics such as polyester resin, polystyrene, polyethylene resin, polypropylene resin, ABS (acrylonitrile-butadiene-styrene) resin, nylon, acrylic resin, fluororesin, polycarbonate resin, polyurethane resin, methylpentene resin, phenolic resin, melamine resin, epoxy resin, and vinyl chloride resin; and agarose, dextran, cellulose, polyvinyl alcohol, nitrocellulose, chitin, and chitosan. The shape of the solid support is also not particularly limited and may be a flat plate, a film, a tube, a particle, and so forth. Such particles include metal beads and magnetized or magnetizable magnetic beads. For example, the target nucleic acid may be covalently bonded to an alkynylated magnetic bead by a click reaction.

In pyrosequencing, dATP, which is structurally similar to ATP, acts as a substrate for luciferase and produces luminescence, and as a result the analytical sensitivity may be reduced by the admixture of dATP. However, the present invention, which uses the mass of the pyrophosphoric acid as an indicator, does not utilize the luciferin-luciferase reaction and the reduction in analytical sensitivity as in pyrosequencing is not produced. However, it is preferable to wash off unreacted substrates, nucleic acids, and so forth as appropriate.

The primer that serves as the starting point for the base extension reaction can be prepared by a production method known to the person skilled in the art, such as chemical synthesis. The primer may as necessary have one or a plurality of labels. Preferred labels can be exemplified by enzymes, biotin, fluorescent substances, haptens, antigens, antibodies, radioactive substances, luminophores, and the like.

The base extension reaction may be a singleplex assay that uses a single primer set to amplify only the target nucleic acid, or may be a multiplex assay that uses a plurality of primer sets to simultaneously amplify a plurality of target nucleic acids or the target nucleic acid and one or a plurality of nucleic acids other than the target nucleic acid.

Multiplex assays are known to the person skilled in the art. πCode technology is a multiplex assay technology; this is a technology that identifies the type of detection target by fixing an antibody, or a probe for gene measurement, to a magnetic microdisc having a barcode engraved on its surface, thereby enabling simultaneous measurement of multiple items. πCode technology is disclosed, for example, in WO 2016/198954 (Japanese Translation of PCT Application No. 2018-518146) and WO 2018/052464 (Japanese Translation of PCT Application No. 2019-535003), and the contents of these publications are incorporated in this Description in their entirety by reference.

The primers used in the base extension reaction may be a primer set that combines a forward primer corresponding to a region located upstream from the target nucleic acid and a reverse primer corresponding to a region located downstream from the target nucleic acid. The length of the primers can be adjusted as appropriate by the person skilled in the art and will presumably be, for example, a length of about 15 to 30 bases.

The target nucleic acid and primer may be preliminarily annealed in an annealing buffer. The resulting double-stranded DNA may be contacted with, e.g., a polymerase, as a pre-annealed heterodimer, without or without being bonded to a support.

A multiplexing that can simultaneously detect a plurality of gene mutations is made possible by designing primer sets that can detect various genes and their gene mutations. These mutations also include epigenetic changes.

A gene mutation can be easily detected when a primer set is prepared that can amplify a region comprising the gene mutation to be detected. Since this has a level of expandability that enables facile amplification of a target gene mutation, a companion diagnostic can be rapidly elaborated also in the event of the emergence of a novel target gene mutation to be detected.

The buffer used in the annealing and base extension reaction is not particularly limited, but is preferably a buffer used in PCR, such as Tris-HCI, which provides a chemical environment adapted for polymerase activity. The pH of the buffer may be 6.0 to 9.5, for example 7.0 to 9.0.

In the base extension reaction, a complex is formed by binding of the polymerase to the target nucleic acid to which the primer has bound. When a substrate such as dNTP binds to this complex and the polymerase fingers close, a three-dimensional structure is formed that comprises the polymerase, the target nucleic acid to which the primer is bound, and the substrate. A phosphodiester bond is formed between the 3'-OH group at the end of the primer strand and the α-phosphorus atom of the substrate, and the bond between the α-phosphorus atom and the β-phosphorus atom of the substrate is cleaved. As a result, the complementary strand is extended by just one base and pyrophosphoric acid is released; since the substrate is labeled with an isotope with a different mass for each base, the released pyrophosphoric acid has a different mass for each base.

The analytic method according to the present invention, which uses pyrophosphoric acid as its indicator, can be employed in various applications. For example, real-time quantitative analysis can be carried out by monitoring, using a mass spectrometer, the pyrophosphoric acid released in the polymerase-mediated base extension reaction. For example, the presence of released pyrophosphoric acid can be used as an indicator of the presence of a target nucleic acid, and, by identifying the bases for the released pyrophosphoric acids, all or part of the base sequence constituting the target nucleic acid can be determined. For example, each one of the four species of dNTPs may be added to the reaction solution in sequence, and the base species of a target site in the target nucleic acid can be determined by detecting the presence of released pyrophosphoric acid only when a nucleic acid base species complementary to the target site in the target nucleic acid is added.

DNA polymerase extends a DNA strand in the presence of the four nucleotide species (dNTPs: dATP, dTTP, dGTP, dCTP). When a DNA strand extension reaction is carried out in the presence of ddNTP (2',3'-dideoxynucleotides: ddATP, ddTTP, ddGTP, ddCTP), the DNA extension reaction stops when a ddNTP is incorporated. This makes it possible to obtain DNA fragments of various strand lengths, which have different lengths for each single base. A ddNTP is incorporated at the 3'-terminal of these DNA fragments. If the ddATP, ddTTP, ddGTP, and ddCTP are labeled with respectively different isotopes, the ddNTP incorporated into the 3'-terminal of a DNA fragment can be identified by identifying the isotope. Sequence analysis can be carried out by reading the type of ddNTP incorporated at the 3'-terminal.

In an embodiment, the analytic method further comprises a step of subjecting a control nucleic acid to steps 1) and 2) and comparing the masses of the released pyrophosphoric acid per one substrate incorporated into the target nucleic acid and the control nucleic acid.

A target nucleic acid and control nucleic acid of any origin may be compared. Thus, the target nucleic acid and the control nucleic acid may not have the same origin and may have different origins. Describing as an example the case in which the target nucleic acid and the control nucleic acid are derived from different subjects, one may be a healthy individual having a wild-type sequence and the other may be a patient having a certain mutant sequence. Which sequence is to be the target nucleic acid or the control nucleic acid depends on the application of the analytic method and can be determined as appropriate by the person skilled in the art.

In an embodiment, when the mass of the released pyrophosphoric acid deriving from an extension reaction of a target nucleic acid is different from that of the released pyrophosphoric acid deriving from an extension reaction of a control nucleic acid, it is then determined that the complementary base for the base incorporated into the target nucleic acid is different from the base of the control nucleic acid at the corresponding position.

When the mass of the released pyrophosphoric acid obtained by an extension reaction on a target nucleic acid obtained from a specimen derived from a healthy individual who is highly likely to have a wild-type gene, is different from the mass of the released pyrophosphoric acid obtained by an extension reaction on a mutant gene used as the control nucleic acid, the nucleic acid contained in the specimen can be determined to be wild-type by comparing the masses of the two. However, the target nucleic acid and the control nucleic acid provided here are merely examples, and, as mentioned above, the control nucleic acid may be a wild-type nucleic acid and the mutant nucleic acid may be the target nucleic acid. Alternatively, both may be mutant nucleic acids with different mutation sites.

Depending on the goal of the analysis of the target nucleic acid, the mass spectrometer can be used in combination with other devices, e.g., electrophoresis instruments, next-generation sequencers, flow cytometers, spectroscopic analysis instrumentation, and so forth.

The analytic method comprises a base extension step (step 1) and a mass spectrometry step (step 2) as essential steps, but may include, before or after each step, for example, after the base extension step, a washing or removal step to remove: suspended synthesis products other than nucleic acids which are on the extension line of an immobilized primer required for the nucleic acid determination process, and excess base that has not been incorporated. Nucleotide-degrading enzymes such as apyrase can be used to remove the substrate. Alternatively, the target nucleic acid can be preliminarily bound to a solid phase and the reaction solution can be washed out to remove excess substrate.

The added nucleotide-degrading enzyme is preferably removed using an ultrafiltration membrane or its activity is stopped by the addition of, e.g., a reagent.

In an embodiment, each step is executed in the analytic method so as to produce an extension reaction one base at a time.

In an embodiment, the analytic method further comprises a step of stopping the extension reaction between steps 1) and 2).

The analytic method may further include, as described in the following, a step of controlling base elongation and a step of evaluating the released pyrophosphoric acid. The present analytic method can be used to detect any base mutation or change, and an example of the instant evaluation method is a method for detecting genetic mutations based on the proportions of different bases, in correspondence to the released pyrophosphoric acid, that are incorporated as reverse complements into the target (template) nucleic acid.

In an embodiment, the abundance ratios of pyrophosphoric acids having different masses and derived from target nucleic acid contained in one or a plurality of specimens is determined with the analytic method.

The proportion of genetic mutations present in a target specimen can be calculated by quantifying the abundance ratios of pyrophosphoric acids released from the four species of substrates. Such base proportions include the abundance ratio of tumor-specific genetic mutations, allele frequency, and mutation proportions such as the methylation ratio of cytosine residues in CpG. In addition, when nucleic acids derived from different types of biological samples, such as cancer cells and normal cells, are contained in a specimen, the abundance ratio of such biological samples in the specimen can be evaluated by using a nucleic acid contained only in one of the biological samples (for example, cancer cells) as the target nucleic acid.

When the analytic method is used for sequence determination, if the mass of the released pyrophosphoric acid derived from the extension reaction of the target nucleic acid differs from that of the released pyrophosphoric acid derived from the extension reaction of the control nucleic acid, the step of determining that the complementary base for the base incorporated at the target nucleic acid is different from the base of the control nucleic acid which is at the corresponding position can be repeated a plurality of times.

The analytic method according to the present invention has elements in common with pyrosequencing, except that the mass of pyrophosphoric acid is used as the indicator. As a consequence, various gene analysis methods based on pyrosequencing are presumed to be examples of the instant evaluation method, for example, analysis of disease-related gene mutations such as single nucleotide polymorphisms (SNPs) and insertions/deletions, quantitative analysis of polymorphisms, quantitative analysis of DNA methylation ratios, microbial identification, detection of drug resistance genes, and so forth.

For example, the allele frequency of the nucleic acid contained in various specimens, e.g., pathogen-infected tissues, tumor biopsies, hematopoietic chimeras, mitochondrial heteroplasmy, and so forth, can be evaluated by comparison to known nucleic acids.

As used in this Description, "allele frequency" refers to the frequency of each allelic gene (allele frequency) in a certain population when multiple alleles exist for a certain gene locus. For example, when each individual has a base sequence of AA, AG, or GG at a position on a certain chromosome, the frequency of A or G in the population to which those individuals belong is referred to as the allele frequency.

When the purpose of the analysis is to detect methylated DNA in a prescribed region or analyze the methylation ratio at particular sites in a tissue, the target nucleic acid is subjected to a base replacement reaction by a bisulfite treatment followed by a determination of its sequence. In such a base replacement reaction, unmethylated cytosine reacts with sodium bisulfite and is converted to uracil, while methylated cytosine does not react with the sodium bisulfite. As a consequence, the DNA methylation ratio can be quantitatively analyzed by converting the difference in DNA methylation (^{m}C/C) into a difference in base sequence (C/T) and carrying out quantitative analysis as a C/T SNP. The analysis according to the present invention can also be applied to the detection of other genetic or epigenetic DNA modifications.

In order to detect a gene, a probe that hybridizes to a specific region of the gene can also be used. An example of such a probe is an oligonucleotide that anneals to a sequence present between the forward primer binding site and the reverse primer binding site. The probe may be labeled, and reporter fluorescent dyes and quencher fluorescent dyes are examples of such a label.

In another embodiment, other enzymes can be brought into contact with the target nucleic acid instead of a polymerase and the pyrophosphoric acid produced as a result can also be detected. For example, AMP-generating DNA ligase or RNA ligase can release pyrophosphoric acid by a process of substrate adenylation.

A ligase is an enzyme that connects two nucleic acids, and any ligase that can release pyrophosphoric acid can be used.

The present invention is described in additional detail in the following using examples, but the present invention is not limited to or by these examples.

### Examples

### 1. Methods

### 1.1. Detection of pyrophosphoric acid by MALDI-TOF/MS

The method for detecting pyrophosphoric acid by MALDI-TOF/MS is described in detain as below.

### 1.1.1. Reagents and apparatus

Sodium pyrophosphate decahydrate (FUJIFILM Wako Pure Chemical Corporation) was used for the pyrophosphoric acid that was the evaluation target. The sodium pyrophosphate decahydrate was prepared at 10 µM using ultrapure water and was diluted as required using ultrapure water.

The following were used for the matrix: DHB (dihydroxybenzoic acid) (FUJIFILM Wako Pure Chemical Corporation), CHCA (α-cyano-4-hydroxycinnamic acid) (Supelco Inc.), IAA (3-indoleacrylic acid) (Sigma), and 9-AA (9-aminoacridine) (Supelco Inc.). In accordance with the pyrophosphoric acid concentration, the matrix was dissolved and its concentration was adjusted to support mixing so as to provide 1,000X to 100,000X relative to the pyrophosphoric acid.

The following solvents were used for the matrices.
DHB, CHCA: 1 : 1 mixed solution of acetonitrile and 0.1% trifluoroacetic acid solution
9-AA: ethanol
IAA: methanol

TFA (trifluoroacetic acid) (Supelco Inc.) or ammonium citrate (DAC) (Sigma) was added at the same time as the ionization aid.

A MALDI-TOF/MS (JMS-S3000 SpiralTOF, JEOL Ltd.) was used for measurement of the pyrophosphoric acid.

### 1.1.2. Sample preparation and measurement

The sample used for measurement was prepared by mixing the particular concentration of the aqueous pyrophosphoric acid solution and the aqueous matrix solution at 1 : 1 as the amount of solution. TFA or ammonium citrate was also added at the same time here as the ionization aid.

0.5 µL of the sample was dripped onto a MALDI-TOF/MS measurement plate (disposable, 384 well plate) and was air dried. Air drying was followed by the MALDI-TOF/MS measurement.

### 1.1.3. Measurement of pyrophosphoric acid using an IAA or 9-AA matrix

The sample was made by mixing 30 mM or 3 mM IAA with 10 µM pyrophosphoric acid and also admixing DAC. The sample containing 9-AA was prepared by mixing 30 mM or 3 mM 9-AA with 10 µM pyrophosphoric acid.

Measurement of the sample by MALDI-TOF/MS confirmed m/z = vicinity of 177, which is the peak for pyrophosphoric acid (Figures 1 and 2). It was also confirmed here that a peak in the vicinity of m/z = 177 was not present for the sample to which pyrophosphoric acid had not been added, and the peak in the vicinity of m/z = 177 was thus identified as the pyrophosphoric acid that was the target of the measurement.

### 1.2. Verification of the pyrophosphoric acid detection sensitivity of MALDI-TOF/MS

### 1.2.1. Sample preparation

The samples used for the measurements were prepared by diluting pyrophosphoric acid in eight steps (100 nM, 10 nM, 1 nM, 100 pM, 10 pM, 1 pM, 100 fM) from 1 µM to 100 fM using a common ratio of 10:1 and mixing with 3,000X 9-AA as the molar ratio to prepare a sample at each pyrophosphoric acid concentration.

### 1.2.2. Sample measurements

Measurement of the samples by MALDI-TOF/MS confirmed the pyrophosphoric acid peak in the vicinity of m/z = 177. At the same time, it was confirmed that the peak in the vicinity of m/z = 177 was absent for samples to which pyrophosphoric acid had not been added, and the peak in the vicinity of m/z = 177 was thus confirmed to be the pyrophosphoric acid measurement target.

### 1.3. Confirmation of detection of pyrophosphoric acid in extension reaction solutions

### 1.3.1. Sample preparation

A reaction solution with the following composition was prepared for the single base extension reaction solution.

**[Table 1]**

| | |
|---|---|
| 10 mM | Tris-HCI (pH 8.0) |
| 50 mM | KCI |
| 1.5 mM | MgCl₂ |
| 100 nM | Primer |
| 100 nM | Template DNA |
| 1.25 U | DNA Polymerase |

The aqueous pyrophosphoric acid solution is added to the extension reaction solution so as to provide a final concentration of 10 µM. The sample was prepared by the addition of 3,000X 9-AA as the molar ratio.

### 1.3.2. Sample measurement

The sample was measured by MALDI-TOF/MS and the pyrophosphoric acid peak residing in the vicinity of m/z = 177 was confirmed. At the same time, it was confirmed that the peak in the vicinity of m/z = 177 was absent for a sample to which pyrophosphoric acid had not been added, and the peak in the vicinity of m/z = 177 was thus confirmed to be the pyrophosphoric acid measurement target.

### 1.4. Investigation of factors influencing ion suppression

### 1.4.1. Sample preparation (for evaluation of the effect of KCI)

With reference to the single base extension reaction solution given in 1.3.1, a reaction solution with the following composition was prepared omitting the KCI.

**[Table 2]**

| | |
|---|---|
| 10 mM | Tris-HCl (pH 8.0) |
| 1.5 mM | MgCl₂ |
| 100 nM | Primer |
| 100 nM | Template DNA |
| 1.25 U | DNA Polymerase |

The aqueous pyrophosphoric acid solution was added to the extension reaction solution so as to provide a final concentration of 10 µM. The sample was prepared by the addition of 3,000X 9-AA as the molar ratio.

### 1.4.2. Sample preparation (for evaluation of the effect of MgCl₂)

With reference to the single base extension reaction solution given in 1.3.1, reaction solutions were prepared that omitted the KCI and contained MgCl₂ so as to provide a final concentration of 1.5 mM, 150 µM, 15 µM, or 1.5 µM; these reaction solutions were used as the samples for the determination of the effect of MgCl₂.

**[Table 3]**

| | |
|---|---|
| 10 mM | Tris-HCI (pH 8.0) |
| 1.5 mM, 150 µM, 15 µM, 1.5 µM | MgCl₂ |
| 100 nM | Primer |
| 100 nM | Template DNA |
| 1.25 U | DNA Polymerase |

The aqueous pyrophosphoric acid solution was added to the extension reaction solution so as to provide a final concentration of 10 µM. The sample was prepared by the addition of 3,000X 9-AA as the molar ratio.

1.4.3. Sample preparation (for evaluation of the effect of Mg ion)

With reference to the single base extension reaction solution given in 1.3.1, a reaction solution was prepared that omitted the KCI and contained MgCl₂ so as to provide a final concentration of 1.5 mM, and a reaction solution was prepared that omitted the KCI and contained MgSO₄ so as to provide a final concentration of 2.0 mM; these reaction solutions were used as the samples for the evaluation of the effect of the Mg ion.

**[Table 4]**

| | | | | |
|---|---|---|---|---|
| 10 mM | Tris-HCI (pH 8.0) | | 10 mM | Tris-HCI (pH 8.0) |
| 1.5 mM | MgCl₂ | | 2.0 mM | MgSO₄ |
| 100 nM | Primer | | 100 nM | Primer |
| 100 nM | Template DNA | | 100 nM | Template DNA |
| 1.25 U | DNA Polymerase | | 1.25 U | DNA Polymerase |

The aqueous pyrophosphoric acid solution was added to the extension reaction solution so as to provide a final concentration of 10 µM. The sample was prepared by the addition of 3,000X 9-AA as the molar ratio.

### 1.4.4. Sample preparation (for evaluation of the effect of the buffer component)

With reference to the single base extension reaction solution given in 1.3.1, reaction solutions were prepared that omitted the KCI and that used MOPS or NaHCO₃ instead of Tris-HCI, and these reaction solutions were used as the samples for evaluating the effect of the buffer component.

**[Table 5]**

| | | | | |
|---|---|---|---|---|
| 50 mM | MOPS (pH 8.0) | | 10 mM | NaHCO₃ (pH 7.9) |
| 1.5 mM | MgCl₂ | | 1.5 mM | MgCl₂ |
| 100 nM | Primer | | 100 nM | Primer |
| 100 nM | Template DNA | | 100 nM | Template DNA |
| 1.25 U | DNA Polymerase | | 1.25 U | DNA Polymerase |

The aqueous pyrophosphoric acid solution was added to the extension reaction solution so as to provide a final concentration of 10 µM. The sample was prepared by the addition of 3,000X 9-AA as the molar ratio.

### 1.4.5. Sample measurements

The samples were measured by MALDI-TOF/MS and the pyrophosphoric acid peak residing in the vicinity of m/z = 177 was confirmed. At the same time, it was confirmed that the peak in the vicinity of m/z = 177 was absent for samples to which pyrophosphoric acid had not been added, and the peak in the vicinity of m/z = 177 was thus confirmed to be the pyrophosphoric acid measurement target.

### 1.5. Investigation into avoiding the effect by changing the metal ion

### 1,5.1 Sample preparation (for evaluation of metal ion effect)

With reference to the single base extension reaction solution given in 1.3.1, reaction solutions were prepared that omitted the KCI and that each contained one of the three metal ions from MgCl₂, MnCl₂, and CoCl₂ at a final concentration of 150 µM, and these reaction solutions were used as the samples for evaluating the effect of the metal ion.

**[Table 6]**

| | | | | |
|---|---|---|---|---|
| 10 mM | Tris-HCI (pH 8.0) | | 10 mM | Tris-HCI (pH 8.0) |
| 150 µM | MgCl₂ | | 2.0 mM | MgSO₄ |
| 100 nM | Primer | | 100 nM | Primer |
| 100 nM | Template DNA | | 100 nM | Template DNA |
| 1.25 U | DNA Polymerase | | 1.25 U | DNA Polymerase |

**[Table 7]**

| | |
|---|---|
| 10 mM | Tris-HCI (pH 8.0) |
| 150 µM | CoCl₂ |
| 100 nM | Primer |
| 100 nM | Template DNA |
| 1.25 U | DNA Polymerase |

Reaction solutions were also prepared by mixing KCI, so as to provide a final concentration of 50 mM, into these three reaction solutions. The aqueous pyrophosphoric acid solution was added to the extension reaction solution so as to provide a final concentration of 10 µM. The sample was prepared by the addition of 3,000X 9-AA as the molar ratio.

### 1.5.2. Sample measurements

The samples were measured by MALDI-TOF/MS and the pyrophosphoric acid peak residing in the vicinity of m/z = 177 was confirmed. At the same time, it was confirmed that the peak in the vicinity of m/z = 177 was absent for samples to which pyrophosphoric acid had not been added, and the peak in the vicinity of m/z = 177 was thus confirmed to be the pyrophosphoric acid measurement target.

1.6. Release of pyrophosphoric acid by a single base extension reaction

Pyrophosphoric acid, which is the target substance measured by MALDI-TOF/MS, is released from DNA by the DNA polymerase-mediated extension reaction. Due to this, conditions are sought under which only a single base extension can occur when the extension reaction is carried out.

### 1.6.1. Preparation of DNA for confirmation of the extension reaction (preparation of template DNA-primer complex)

The following artificially synthesized oligo-DNAs were prepared as DNA for confirmation of the extension reaction.

**[Table 8]**

| DNA | Sequence | Lengt h |
|---|---|---|
| template-polyC-Bio | GCGCGGGACTGGCCGTCGTTTTACAAGCCGCCGCC GCCGAACCGACAC/3Bio/ (SEQ ID NO: 1) | 48 mer |
| template-3nt | GGGACTGGCCGTCGTTTTACAAGCCGCCGCCGCCGA ACCGACAC（SEQ ID NO: 2） | 44 mer |
| AF647-primer-21nt | /AF647/TTGTAAAACGACGGCCAGTCC (SEQ ID NO: 3 ) | 21 mer |

| | | |
|---|---|---|
| Bio : biotin label AF647 : AF647 fluorescent label | | |

The template DNA-primer complexes were mixed in the following two combinations.

**[Table 9]**

| ID | Biotin-Modified Template DNA | Strand Length | AF647-Labeled Primer | Strand Length |
|---|---|---|---|---|
| HD3 | template-polyC-Bio | 48 mer | AF647-primer-21 nt | 21 mer |
| HD5 | template-3nt | 44 mer | AF647-primer-21nt | 21 mer |

These two combinations were mixed in the following reaction solution for complex preparation.

**[Table 10]**

| Reaction Solution for Complex Preparation | Final Concentration |
|---|---|
| Biotin-Modified Template DNA | 10 µM |
| AF647-Labeled Primer | 10 µM |
| 10×ThermoPol Buffer | 1× |
| Ultrapure Water | |

| | |
|---|---|
| The 10× ThermoPol Buffer contains: 200 mM Tris-HCI (pH 8.8), 100 mM (NH₄)₂SO₄, 100 mM KCI, 20 mM MgSO₄, and 1% Triton X-100. | |

The following reaction was carried out in a thermal cycler: "98°C/1 minute, 80°C/10 minutes → temperature reduced from 80°C to 20°C in 0.5°C steps; reaction was carried out at each temperature for 1 minute".

The solution after the completion of the reaction was designated the 10 µM template DNA-primer complex.

### 1.6.2. Preparation of extension reaction solution and extension reaction (investigation using MgCl₂)

The following extension reaction solutions were prepared.

**[Table 11]**

| Extension Reaction Solution | Final Concentration |
|---|---|
| Ultrapure Water | |
| 10×PCR Buffer (Mg²⁺ free) | 1× |
| MgCl₂ | 150 µM |
| dNTP | 2 µM |
| ddNTP | 500, 200, 100, 50, 10, 5, 1 µM |
| DNA Polymerase β | 22.5 U |

| | |
|---|---|
| The 10× PCR Buffer (Mg²⁺ free) contains the following: 100 mM Tris-HCI (pH 8.9), 500 mM KCI. | |

The template DNA-primer complex was added to the reaction solution so as to provide a final concentration of 50 nM, and the extension reaction was run by reacting for 2 minutes/37°C in a thermal cycler.

### 1.6.3. Preparation of extension reaction solutions and extension reactions (investigations using MnCl₂ and CoCl₂)

The following extension reaction solutions were prepared.

**[Table 12]**

| Extension Reaction Solution | Final Concentration |
|---|---|
| Ultrapure Water | |
| 10×PCR Buffer (Mg²⁺ free) | 1× |
| MnCl₂, CoCl₂ | 150, 15, 1.5 µM |
| dNTP | 20, 2 µM |
| ddNTP | 500, 200, 20, 2 µM |
| DNA Polymerase β | 22.5 U |

| | |
|---|---|
| The 10× PCR Buffer (Mg²⁺ free) contains the following: 100 mM Tris-HCI (pH 8.9), 500 mM KCI. | |

The template DNA-primer complex was added to the reaction solution so as to provide a final concentration of 50 nM, and the extension reaction was run by reacting for 2 minutes/37°C in a thermal cycler.

### 1.6.4. Confirmation of extension reaction using urea-modified polyacrylamide gel electrophoresis

A urea-modified polyacrylamide gel with the following composition was prepared.

**[Table 13]**

| Urea-Modified Polyacrylamide Gel | Final Concentration |
|---|---|
| Ultrapure Water | |
| 40% Acrylamide/Bisacrylamide Mixture | 20% |
| 10×TBE Solution | 1× |
| Urea | 7M |
| Ammonium Persulfate | 0.5% |
| N, N, N', N'- Tetramethylethylenediamine | 0.1% |

The extension reaction solution was applied to the urea-modified polyacrylamide gel and electrophoreses was performed. The extension reaction was confirmed by staining the DNA with SYBR Gold and imaging the gel with a camera.

### 2. Results and Discussion

### 2.1. Detection of pyrophosphoric acid by MALDI-TOF/MS

As a result of measurements using DHB or CHCA as the matrix, m/z = 159 was obtained, which is a peak less than pyrophosphoric acid, and it was thought that HP₂O₆ was obtained, which is smaller than the desired H₃P₂O₇, which is the pyrophosphoric acid skeleton. Since it was thought that fragmentation with DHB or CHCA was strong, the measurements were repeated using IAA or 9-AA, with which fragmentation is milder.

As a result, a peak in the vicinity of m/z = 177 was obtained, which was thought to be the target H₃P₂O₇. In this case, the target peak was obtained with IAA when DAC was used as an ionization aid, while with 9-AA the target peak was obtained even without an ionization aid. Therefore, the matrices suitable for detecting pyrophosphoric acid were IAA or 9-AA, which provide mild fragmentation, and the target peak was obtained with 9-AA even without an ionization aid. 9-AA was used in the ensuing studies since 9-AA does not require an ionization aid.

### 2.2. Confirmation of pyrophosphoric acid detection sensitivity by MALDI-TOF/MS

According to the results of the measurements, the target peak could be confirmed to and including 1 pM. The detection sensitivity of test methods for measuring gene mutations is generally 10 pM, and it could thus be shown that the pyrophosphoric acid detection method using MALDI-TOF/MS has a sensitivity equal to or better than that of existing methods.

2.3. Confirmation of the detection of pyrophosphoric acid in the extension reaction

The pyrophosphoric acid that is the target substance of the measurement requires measurement of the pyrophosphoric acid released by a DNA polymerase-mediated extension reaction. As a consequence, the detectability of the pyrophosphoric acid in the DNA polymerase extension reaction solution was checked. As a result, it could be confirmed that the peak intensity was reduced 1,000-fold. Ions such as K⁺, Mg²⁺, and CI⁻ were present in the reaction solution, and it was presumed that these had an influence in the form of an ion suppression effect when pyrophosphoric acid is measured by MALDI-TOF/MS. The identification of the substances that influenced ion suppression was therefore performed.

### 2.4. Investigation of factors that influence ion suppression

### 2.4.1. Sample preparation (for evaluation of the effect of KCI)

The influence of ion suppression was evaluated through the presence/absence of KCI in the extension reaction solution. KCI is a substance that affects the accuracy of base incorporation during the extension reaction. The peak intensity was improved 10-fold as a result of the removal of KCI, which confirmed that KCI acts via ion suppression. However, other compositions were also evaluated since the improvement was only 10-fold.

### 2.4.2. Sample preparation (for evaluation of the effect of MgCl₂)

The effect of MgCl₂, which like KCI contains CI⁻, was evaluated. As a result of evaluation at the four concentrations of 1.5 mM and 150, 15, and 1.5 µM, a concentration-dependent ion suppression was confirmed. Since the ion suppression effect was strong for the two species, i.e., KCI and MgCl₂, it was thought that CI⁻ was the substance causing the ion suppression effect. On the other hand, since MgCl₂ is a substance essential for base extension, investigations were carried out into whether the effect of CI⁻ could be avoided by replacement with another Mg ion-containing substance.

### 2.4.3. Sample preparation (for evaluation of the effect of Mg ion)

Since MgSO₄ is used as an Mg ion in DNA polymerase extension reactions, the ion suppression effect was checked changing over to MgSO₄. According to the results, MgSO₄ had an even greater ion suppression effect than MgCl₂. This was thought to be due to the strong effect of SO₄²⁻, and it was thus determined that changing to MgSO₄ was not appropriate.

### 2.4.4. Sample preparation (for evaluation of the effect of the buffer component)

Considering the influence of CI⁻, the buffer used in the extension reaction was changed in order to avoid the effect of the CI⁻ in the Tris-HCI buffer component. An investigation was carried out using MOPS, which has been reported for extension reactions, and NaHCO₃, which has a buffering action at around pH 8. According to the results, the peak intensity was highest with Tris-HCI, and MOPS and NaHCO₃ were therefore not suitable for the herein described method.

### 2.5. Investigation into avoiding the effect by changing the metal ion

Considering further that the concentration-dependent ion suppression from MgCl₂ is not strongly influenced by CI⁻ while Mg²⁺ has a strong effect, a metal ion without SO₄²⁻ that can be used in extension reactions was sought. MgCl₂ is generally known to exhibit a strong extension activity and a high base incorporation accuracy, but the herein described method employs a reaction system adequate for the extension of only one to several bases and it is acceptable even if the extension activity and accuracy are not high. Mn²⁺ and Co²⁺, being metal ions that are active with a wide range of DNA polymerase species, were therefore selected, and MnCl₂ or CoCl₂ was used as a substance used in the extension reaction. According to the results, it could be confirmed that the ion suppression effect was lower than that of MgCl₂, which suggested that the effect can be avoided by using MnCl₂ or CoCl₂.

The effect of MnCl₂ or CoCl₂ in the presence of KCI, which acts in ion suppression, was also evaluated. According to the results, it was confirmed that the effect of ion suppression can be avoided with MnCl₂ or CoCl₂.

### 2.6. Release of pyrophosphoric acid by a single base extension reaction

### 2.6.1. Investigation of an extension reaction of only a single base using MgCl₂

The herein disclosed method provides a method that brings about the extension reaction of only a single base using ddNTP, causing the release of pyrophosphoric acid for the single base, and that analyzes this pyrophosphoric acid by mass spectrometry. On the other hand, when attempting to bring about the extension reaction of only a single base using only ddNTP, the DNA polymerase cannot incorporate ddNTP, and as a consequence the extension reaction is enabled by the co-presence of dNTP in the reaction solution. Therefore, reaction conditions for only single base extension were found while also adding dNTP and not just ddNTP.

According to the results for the extension reactions, the condition bringing about an extension reaction of only a single base was found when ddNTP : dNTP mixing ratio = 500 : 1. In addition, by making the template DNA side only one base longer than the primer, an extension reaction of only a single base could also be brought about.

### 2.6.2. Investigation of an extension reaction of only a single base using MgCl₂

The single base extension reaction when using MnCl₂ or CoCl₂, which can avoid the ion suppression effect, was then checked. According to the results, an extension reaction of only a single base could be observed under the conditions shown in the table below.

**[Table 14]**

| dNTP | 20 µM | | | | 2 µM | | | |
|---|---|---|---|---|---|---|---|---|
| ddNTP | 500 µM | 200 µM | 20 µM | 2 µM | 500 µM | 200 µM | 20 µM | 2 µM |
| 150 µM MnCl₂ | | | | | | ✔ | | |
| 15 µM MnCl₂ | ✔ | ✔ | | | ✔ | ✔ | | |
| 1.5 µM MnCl₂ | | ✔ | | | ✔ | ✔ | ✔ | |
| 150 µM CoCl₂ | ✔ | | | | ✔ | ✔ | | |
| 15 µM CoCl₂ | ✔ | ✔ | | | ✔ | ✔ | | |
| 1.5 µM CoCl₂ | | ✔ | | | ✔ | ✔ | ✔ | |

In view of the results reported above, appropriate concentrations of dNTP, ddNTP, MnCI₂, and CoCl₂ that enable only a single base extension, while avoiding the ion suppression effect, could be elucidated for the measurement of the released pyrophosphoric acid by MALDI-TOF/MS using MnCl₂ or CoCl₂ as the metal ion required for the DNA polymerase-mediated extension reaction.

Accordingly, it could be confirmed that, by combining these conditions, the pyrophosphoric acid released by a single base extension could be measured using MALDI-TOF/MS and the pyrophosphoric acid derived from a specific base could be detected.

## Claims

1. A method for detecting pyrophosphoric acid in a sample, the method comprising:
a step of ionizing pyrophosphoric acid derived from a phosphoric acid compound in a sample and determining mass of this pyrophosphoric acid by matrix-assisted laser desorption/ionization mass spectrometry (MALDI/MS).

2. The method according to claim 1, wherein the ionization is carried out in the presence of a matrix.

3. The method according to claim 2, wherein the matrix comprises one or a plurality of cyclic compounds.

4. The method according to claim 3, wherein the cyclic compound has a heterocycle.

5. The method according to claim 4, wherein a heteroatom constituting the heterocycle is nitrogen.

6. The compound according to claim 3, wherein the cyclic compound is a compound selected from the group consisting of trans-3-indoleacrylic acid (IAA), 9-aminoacridine (9-AA), 9H-pyrido[3,4-b]indole, and 1-methyl-7-methoxy-3,4-dihydro-β-carboline.

7. The method according to claim 2, wherein the matrix further comprises an ionization aid.

8. The method according to claim 1 or 2, wherein the sample comprises a nucleic acid as the phosphoric acid compound and a polymerase, and wherein the pyrophosphoric acid released from the nucleic acid by the polymerase is detected.

9. The method according to claim 8, wherein ionization is carried out in the presence of manganese chloride and/or cobalt chloride.
